# EUROPEAN PATENT APPLICATION

(11) **EP 3 892 289 A1**
(43) Date of publication of application: **13.10.2021**
(21) Application number: 19892572.9
(22) Date of filing: 14.11.2019
(51) Int. Cl.: A61K 38/08, A61K 9/00, A61P 1/16

(54) **PHARMACEUTICAL COMPOSITION, COMPRISING INHIBITORY PEPTIDE AGAINST FAS SIGNALING, FOR PREVENTION OR TREATMENT OF OBESITY, FATTY LIVER, OR STEATOHEPATITIS**

(30) Priority: 04.12.2018 KR 20180154274
(71) Applicant: Signet Biotech Inc., Seoul 04763 (KR)
(72) Inventor: LEE, Sang-Kyung, Seoul 04763 (KR); CHUNG, Kunho, Seoul 04763 (KR); ULLAH, Irfan, Seoul 04763 (KR); BAE, Su Min, Seoul 04763 (KR); LIM, Jae Yeoung, Seoul 04763 (KR)
(74) Representative: Dehns
(86) International application number: PCT/KR2019/015585
(87) International publication number: WO 2020/116810

(57) **Abstract**

The present invention relates to a pharmaceutical composition comprising an inhibitory peptide against Fas signaling as an active ingredient for prevention or treatment of obesity, fatty liver, or steatohepatitis. Binding specifically to Fas, which is extensively expressed in the liver and adipose tissues in an obese state, the inhibitory peptide against Fas signaling exhibits high delivery rates to inflammatory regions caused by obesity and directly inhibits the Fas signaling, which is a main inflammation signaling pathway, to inhibit an inflammatory response. Therefore, the peptide can be advantageously used for alleviating or treating obesity, fatty liver, or steatohepatitis.

<110>    Industry-University Cooperation Foundation Hanyang University

<120>    Pharmaceutical composition for preventing or treating of obesity
         or fatty liver comprising Fas signal blocking peptides

<130>    X19U10C0246_O20190328WO

<150>    KR 10-2018-0154274
<151>    2018-12-04

<160>    13

<170>    KoPatentIn 3.0

<210>    1
<211>    5
<212>    PRT
<213>    Artificial Sequence

<220>
<223>    Fas binding peptide


<400>    1
Cys Asp Glu His Phe
  1               5


<210>    2
<211>    6
<212>    PRT
<213>    Artificial Sequence

<220>
<223>    Fas binding peptide


<400>    2
Cys Asp Glu His Phe Tyr
  1               5


<210>    3
<211>    6
<212>    PRT
<213>    Artificial Sequence

<220>
<223>    Fas binding peptide


<400>    3
Cys Asp Glu His Phe Cys
  1               5


<210>    4
<211>    6
<212>    PRT
<213>    Artificial Sequence

<220>
<223>    Fas binding peptide

<400>    4
Cys Asp Glu His Phe Ala
1               5

<210>    5
<211>    7
<212>    PRT
<213>    Artificial Sequence

<220>
<223>    Fas binding peptide

<400>    5
Cys Asp Glu His Phe Cys Tyr
1               5

<210>    6
<211>    7
<212>    PRT
<213>    Artificial Sequence

<220>
<223>    Fas binding peptide

<400>    6
Cys Asp Glu His Phe Ala Tyr
1               5

<210>    7
<211>    6
<212>    PRT
<213>    Artificial Sequence

<220>
<223>    Fas binding peptide

<400>    7
Tyr Cys Asp Glu His Phe
1               5

<210>    8
<211>    7
<212>    PRT
<213>    Artificial Sequence

<220>
<223>    Fas binding peptide

<400>    8
Tyr Cys Asp Glu His Phe Cys
1               5

<210>    9
<211>    7
<212>    PRT
<213>    Artificial Sequence

<220>
<223>    Fas binding peptide


<400>    9
Tyr Cys Asp Glu His Phe Ala
 1               5


<210>    10
<211>    7
<212>    PRT
<213>    Artificial Sequence

<220>
<223>    Fas binding peptide-7 (FBP-7)


<400>    10
Tyr Cys Asp Glu His Phe Tyr
 1               5


<210>    11
<211>    8
<212>    PRT
<213>    Artificial Sequence

<220>
<223>    Fas binding peptide-8 (FBP-8)


<400>    11
Tyr Cys Asp Glu His Phe Cys Tyr
 1               5


<210>    12
<211>    8
<212>    PRT
<213>    Artificial Sequence

<220>
<223>    Fas binding peptide-A (FBP-A)


<400>    12
Tyr Cys Asp Glu His Phe Ala Tyr
 1               5


<210>    13
<211>    8
<212>    PRT
<213>    Artificial Sequence

<220>
<223>    control peptide

<400>    13
Tyr Cys Asn Ser Thr Val Cys Tyr
1               5

## Description

### [Technical Field]

The present invention relates to a pharmaceutical composition comprising an inhibitory peptide against Fas signaling as an active ingredient for prevention or treatment of obesity, fatty liver, or steatohepatitis.

### [Background Art]

Obesity is spreading very rapidly worldwide, and currently, 1.7 billion people corresponding to 25% of the world's population are overweight (BMI of25 or more), and the number of obese patients with a BMI of 30 or more amount to about 300 million people in Western Europe. Further, one out of five children falls under childhood obesity, and the number is increasing rapidly such that childhood obesity has emerged as a serious social problem. Particularly, childhood obesity may cause growth disorders such as precocious puberty because the more fat a child has, the more stimulated the secretion of sex hormones is, and childhood obesity is also responsible for growth inhibition by affecting blood circulation and nutritional balance. Obesity is involved in the development of adult diseases such as hypertension, diabetes, arteriosclerosis, stroke, heart attacks and various tumors as risk factors for major adult diseases (Wilson et al., 2005, Circulation 112: 3066-3072), and also promotes the progression of the disease, and the risk of developing an adult disease due to obesity is known to be 3- to 6-fold higher than that of normal people. Therefore, obesity is not just a cosmetic problem, but is a serious problem that is directly related to health.

In particular, while the obese population is increasing, the number of patients with non-alcoholic fatty liver, in which the energy remaining in the body is accumulated in the form of triglycerides in the liver, is also increasing. When non-alcoholic fatty liver is prolonged, an inflammatory response is induced and progresses to steatohepatitis, and may eventually worsen to cirrhosis and liver cancer, so non-alcoholic fatty liver needs to be prevented and effectively treated.

Novo Nordisk's Saxenda (ingredient name liraglutide), which is currently used as a therapeutic agent for obesity, is a GLP-1 analog, is about 97% similar to the human hormone GLP-1, and is subcutaneously administered once per day. Clinical studies reveal that Saxenda can obtain many health benefits including improvement in blood sugar levels and blood pressure, cholesterol levels, and obstructive sleep apnea syndrome by reducing the body weight of an obese person is reduced by 5 to 10 kg regardless of his or her initial body weight and when the reduced body weight is maintained. However, there are side effects such as nausea, vomiting, diarrhea, and constipation, and even for those who have succeeded in losing weight, discontinuation of administration of the drug leads to the yoyo phenomenon (a phenomenon in which the body weight returns to its original value), so obesity cannot be fundamentally treated without improvement in lifestyles.

As a result of research for the fundamental treatment of obesity and fatty liver, the present inventors found that a Fas receptor is extensively expressed not only in adipose tissues, but also in the liver, and confirmed that obesity, fatty liver, or steatohepatitis can be alleviated by blocking the signaling of the Fas receptor to inhibit an inflammatory response, thereby completing the present invention.

### [Disclosure]

### [Technical Problem]

An object of the present invention provides an inhibitory peptide against Fas signaling and a pharmaceutical composition comprising the peptide as an active ingredient for prevention or treatment of obesity, fatty liver, or steatohepatitis.

### [Technical Solution]

To achieve the object, an aspect of the present invention provides a pharmaceutical composition comprising an inhibitory peptide against Fas signaling, comprising an amino acid sequence represented by the following General Formula 1 as an active ingredient for prevention or treatment of obesity, fatty liver, or steatohepatitis:

[General Formula I] Xaai-Cys-Asp-Glu-His-Phe-Xaa₂-Xaa₃

In the general formula,
Xaa₁ and Xaa₃ are each independently absent or any amino acid, and
Xaa₂ is absent or selected from the group consisting of Ala, Gly, Val, Leu, Ile, Met, Pro, Ser, Cys, Thr, Asn, and Gln.

As used herein, the term "Fas" also refers to Fas, a Fas receptor (FasR), apoptosis antigen 1 (APO-1), or a cluster of differentiation 95 (CD95), and is a type of tumor necrosis factor (TNF) receptor that regulates apoptosis. When Fas binds to a ligand, it is activated through multimerization, and as a result, various adaptor proteins bind to Fas. The bound adaptor proteins activate various apoptosis signaling pathways, and representative signaling regulators include caspase, NF-_{K}B, a stress-activated protein kinase (SAPK), the Bcl-2 family, and the like.

As used herein, the term "inhibitory peptide against Fas signaling" refers to a peptide having an activity of binding to Fas as described above to suppress the downstream signaling pathway of Fas, which is contrary to a typical Fas ligand, and an activity of suppressing apoptosis.

In an exemplary embodiment of the present invention, in General Formula I, Xaa₁ and Xaa₃ may each be independently absent or any amino acid, and preferably, may each be independently selected from the group consisting of Tyr, Phe, and Trp.

Further, in General Formula I, Xaa₂ may be absent or selected from the group consisting of Ala, Gly, Val, Leu, Ile, Met, Pro, Ser, Cys, Thr, Asn, and Gln, and preferably may be selected from the group consisting of Gly, Ala, Ser, Thr, and Cys.

For example, various combinations are possible, such as 1) only Xaa₂ may be present while both Xaa₁ and Xaa₃ are absent, 2) Xaa₂ may be present while any one of Xaa₁ and Xaa₃ is present, and 3) all of Xaa₁ to Xaa₃ are absent.

In an exemplary embodiment of the present invention, the amino acid sequence of General Formula I may be a sequence selected from the group consisting of SEQ ID NOS: 1 to 12, preferably may be selected from the group consisting of SEQ ID NOS: 7 to 12, and more preferably, may be a sequence selected from the group consisting of SEQ ID NOS: 10 to 12.

Meanwhile, for the inhibitory peptide against Fas signaling used in the present invention, the N- and/or C-end of the peptide may be modified in order to obtain the improved stability, enhanced pharmacological properties (half-life, absorbability, titer, efficacy, and the like), altered specificity (for example, broad biological activity spectrum), and reduced antigenicity of the peptide. The above formula may be in a form in which an acetyl group, a fluorenyl methoxy carbonyl group, an amide group, a formyl group, a myristyl group, a stearyl group, or polyethylene glycol (PEG) binds to the N- and/or C-end of the peptide, but the modification of the peptide may particularly include any component that can improve the stability of the peptide without limitation. As used herein, the term "stability" refers not only to *in vivo* stability that protects the peptides of the invention from attack of a protein cleaving enzyme *in vivo,* but also to storage stability (for example, room-temperature storage stability).

In the present invention, the obesity includes obesity-related diseases such as diabetes, fatty liver, hyperlipidemia, arteriosclerosis and complications thereof in addition to obesity, and the fatty liver may be non-alcoholic fatty liver.

Non-alcoholic fatty liver is a disease occurring as a result of the accumulation of excess energy in the form of triglycerides in the liver due to poor lifestyle-related habits such as lack of exercise and a high-calorie diet. When non-alcoholic fatty liver is left untreated, it may progress from hepatitis and cirrhosis to liver cancer, but to date, there is no proper therapeutic drug for non-alcoholic fatty liver, so treatment by exercise and dietary improvement is mainly performed. In the present invention, the non-alcoholic fatty liver includes various forms of liver diseases ranging from simple non-alcoholic fatty liver that simply accumulates only fat and causes almost no damage to hepatocytes, chronic non-alcoholic steatohepatitis with severe hepatocellular damage, and liver cirrhosis.

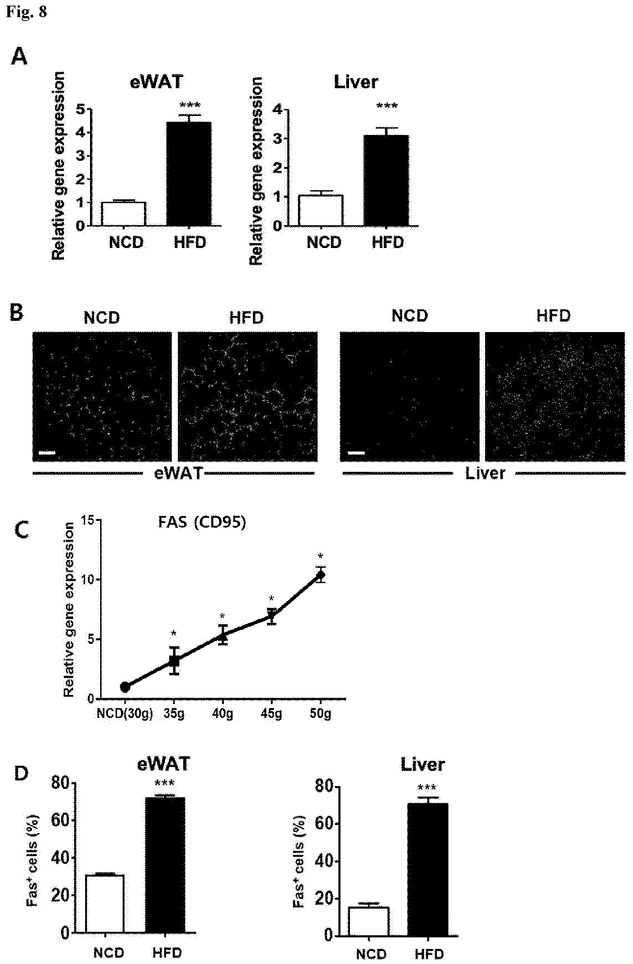

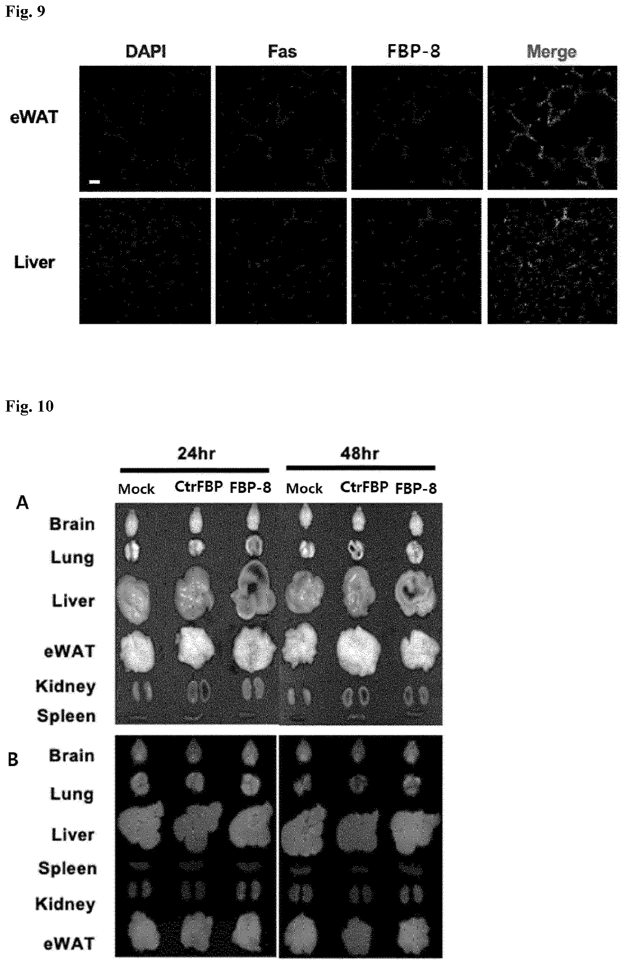

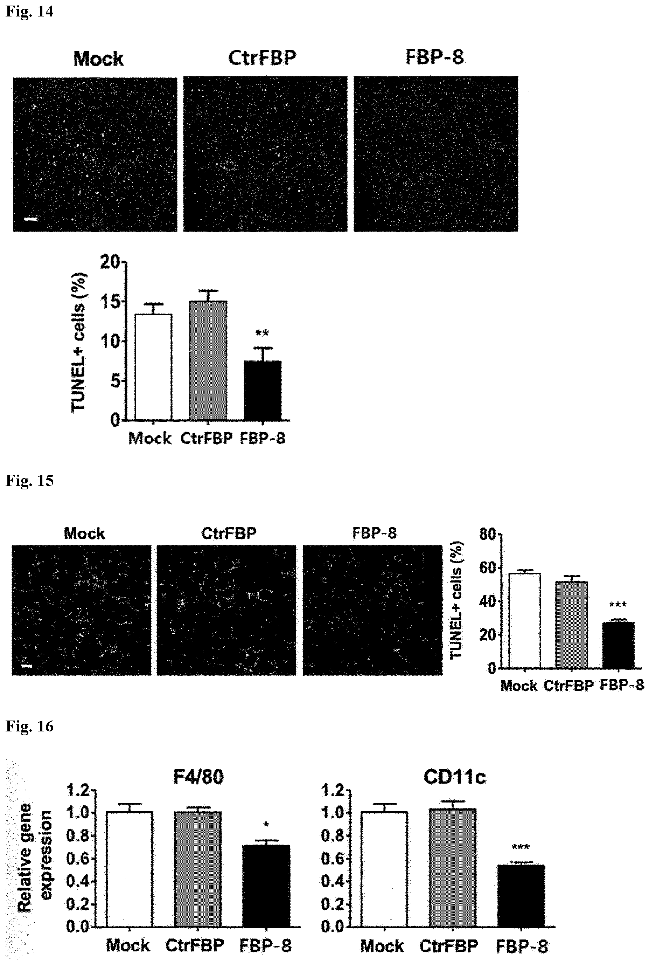

The present inventors confirmed that Fas is extensively expressed not only in adipose tissues but also in liver tissues in an obese state, and in particular, in the liver, the expression of Fas increases proportionally as the obesity level increases (FIG. 8). Thus, it could be seen that when an inhibitory peptide against Fas signaling was administered *in vivo,* the peptide was specifically delivered to the liver and adipose tissue of obesity model mice (FIG. 10) and thus bound to Fas (FIG. 9). That is, it was confirmed that the present invention inhibited an inflammatory response, apoptosis, fat accumulation, and the like due to Fas signaling by directly delivering a drug (an inhibitory peptide against Fas signaling) to adipose tissues and liver tissues, and a result, could improve the symptoms of obesity, fatty liver, or steatohepatitis (FIGS. 14 to 44).

As used herein, the term "treatment" refers to all actions in which symptoms of obesity, fatty liver or steatohepatitis-related diseases are ameliorated or beneficially altered by administration of an inhibitory peptide against Fas signaling according to the present invention or a pharmaceutical composition including the same.

As used herein, the term "administration" refers to introducing a predetermined material, that is, an inhibitory peptide against Fas signaling according to the present invention or a pharmaceutical composition including the same, into a subject, in any appropriate manner. Accordingly, the pharmaceutical composition of the present invention may be administered by a method such as intraperitoneal administration, intravenous administration, intramuscular administration, subcutaneous administration, intradermal administration, oral administration, topical administration, intrapulmonary administration, and rectal administration, but is preferably administered intravenously, subcutaneously or orally. Specifically, when the pharmaceutical composition of the present invention is used for preventing or treating obesity, intravenous administration is preferred, and when the composition is used for preventing or treating fatty liver or steatohepatitis, subcutaneous administration is preferred.

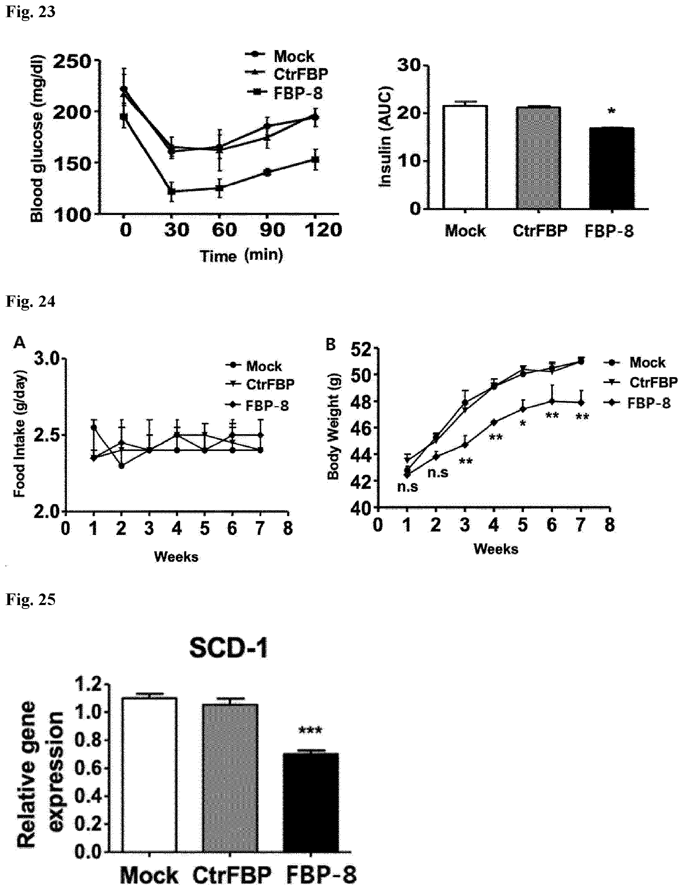

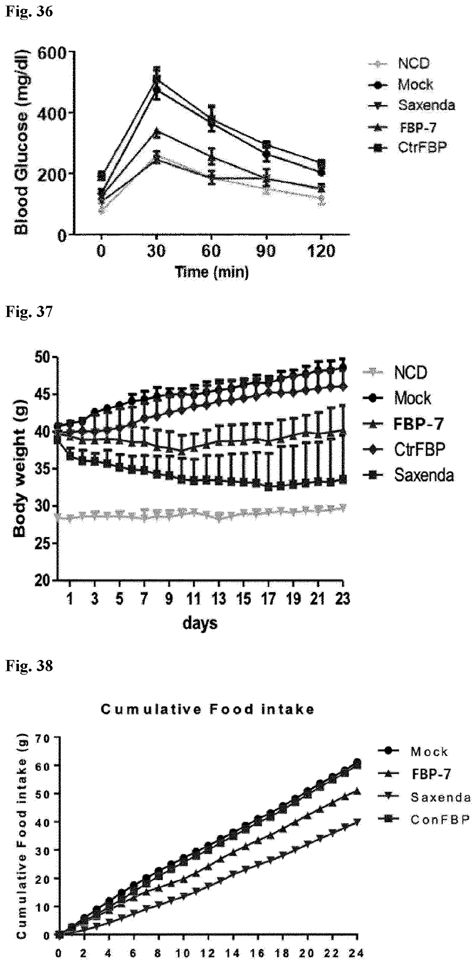

According to an exemplary embodiment of the present invention, the inhibitory peptide against Fas signaling of the present invention is characterized by being specifically delivered to the liver and adipose tissues during intravenous injection (during systemic delivery) and being specifically delivered to the liver during subcutaneous injection (FIG. 10) to inhibit an inflammatory response. Furthermore, when an inhibitory peptide against Fas signaling was administered intravenously to obesity model mice, the body weight increased (FIG. 24A), but the body weight tended to be maintained during subcutaneous injection (FIG. 37). Through this, it can be seen that even when the same amount of an inhibitory peptide against Fas signaling or a pharmaceutical composition including the same is used, the level of treatment/alleviation effect on obesity, fatty liver or steatohepatitis may vary depending on the administration route.

Meanwhile, the pharmaceutical composition of the present invention may be administered in the form of an injection so as to be administered intravenously or subcutaneously. When the pharmaceutical composition of the present invention is prepared as an injection, a buffer, a preservative, a soothing agent, a solubilizing agent, an isotonic agent, a stabilizer, and the like may be mixed, and the pharmaceutical composition of the present invention may be prepared in the form of a unit dose ampoule or a multiple dose.

In the present invention, the "containing as an active ingredient" refers to an amount sufficient to treat a disease at a reasonable benefit/risk ratio applicable to medical treatment, and the level of the effective dosage can be determined according to the type and severity of disease of a patient, the activity of the drug, the drug sensitivity in a patient, the administration time, the administration pathway and release rate, the treatment duration, elements including drugs that are simultaneously used with the composition of the present invention, or other elements well-known in the medical field. The peptide according to the present invention or a pharmaceutical composition including the same may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by the person skilled in the art. The dosage and frequency of the pharmaceutical composition of the present invention are determined by the type of active ingredient, as well as various related factors such as the disease to be treated, the administration route, the age, sex, and body weight of a patient, and the severity of the disease.

Therefore, in addition to including an inhibitory peptide against Fas signaling as an active ingredient, the pharmaceutical composition of the present invention may further include a pharmaceutically acceptable carrier. A pharmaceutically acceptable carrier included in the pharmaceutical composition of the present invention is typically used during formulation, and includes lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, and the like, but is not limited thereto. The pharmaceutical composition of the present invention may additionally contain a lubricant, a wetting agent, a sweetening agent, a flavoring agent, an emulsifier, a suspending agent, a preservative, and the like, in addition to the aforementioned ingredients. Suitable pharmaceutically acceptable carriers and formulations are described in detail in Remington's Pharmaceutical Sciences (19th ed., 1995).

A suitable dose of the pharmaceutical composition of the present invention may vary depending on factors, such as formulation method, administration method, age, body weight, sex or disease condition of the patient, diet, administration time, administration route, excretion rate and response sensitivity. Meanwhile, the dose of the pharmaceutical composition of the present invention is preferably 0.001 to 1000 mg/kg (body weight) daily.

The pharmaceutical composition of the present invention may be prepared in the form of a unit-dose or by being contained in a multi-dose container by being formulated using a pharmaceutically acceptable carrier and/or excipient according to a method that can be readily implemented by a person with ordinary skill in the art to which the present invention pertains. In this case, a dosage form may also be in the form of a solution in an oil or aqueous medium, a suspension or in the form of an emulsion, an extract, a powder, a granule, a tablet or a capsule, and the pharmaceutical composition of the present invention may additionally include a dispersant or a stabilizer.

Another aspect of the present invention provides an inhibitory peptide against Fas signaling, including an amino acid sequence represented by the following General Formula I:

Xaa1-Cys-Asp-Glu-His-Phe-Xaa2-Xaa3

In the general formula,
Xaa1 and Xaa3 are each independently absent or any amino acid, and
Xaa₂ is absent or selected from the group consisting of Ala, Gly, Val, Leu, Ile, Met, Pro, Ser, Cys, Thr, Asn, and Gln.

Since the inhibitory peptide against Fas signaling is the same as an inhibitory peptide against Fas signaling included in a pharmaceutical composition for treatment or amelioration of obesity, fatty liver, or steatohepatitis, the duplicate content thereof will be omitted.

### [Advantageous Effects]

Since the inhibitory peptide against Fas signaling according to the present invention binds specifically to Fas, which is extensively expressed in the liver and adipose tissues in an obese state, the inhibitory peptide against Fas signaling exhibits high delivery rates to inflammatory regions caused by obesity. Moreover, the inhibitory peptide can directly inhibit the Fas signaling, which is a main inflammation signaling pathway, to inhibit an inflammatory response. Therefore, the peptide can be advantageously used for alleviating or treating obesity, fatty liver, or steatohepatitis.

### [Description of Drawings]

FIG. 1 illustrates the results of confirming whether FBP-8, FBP-A, and FBP-7, which are inhibitory peptides against Fas signaling, treated to a Jurkat cell line always expressing Fas bind to Fas: Mock = untreated group (experimental group not treated with any peptide); CtrFBP = negative control (control peptide-treated group); FBP-8 = YCDEHFCY peptide-treated group; FBP-A = YCDEHFAY-treated group and FBP-7 = YCDEHFY-treated group.
FIG. 2 illustrates the results of confirming the levels of apoptosis after treating Jurkat cell lines with a Fas ligand (FasL) and FBP-8, FBP-A, or FBP-7 which is an inhibitory peptide against Fas signaling in combination (A) and confirming the stability of FBP-8 and FBP-7 in sera (B).
FIG. 3 illustrates the results of confirming the presence or absence of cytotoxicity after treating 3T3L1 cell lines with various concentrations of FBP-8.
FIG. 4 illustrates the results of confirming the levels of apoptosis after treating 3T3L1 cell lines with a Fas ligand (FasL) and FBP-8 in combination.
FIG. 5 illustrates the results of confirming the expression levels of inflammatory response-related genes after treating 3T3L1 cell lines with a Fas ligand (FasL) and FBP-8 in combination.
FIG. 6 illustrates the results of confirming the levels of inflammatory response-related cytokines in cell culture solutions after treating 3T3L1 cell lines with a Fas ligand (FasL) and FBP-8 in combination.
FIG. 7 illustrates the results of confirming the concentrations of a free fatty acid (FFA) released into cell culture solutions after treating 3T3L1 cell lines with a Fas ligand (FasL) and FBP-8 in combination.
FIG. 8 illustrates the results of confirming Fas expression levels (A and B) in white adipose tissues and liver tissues of normal body weight mice (NCD), changes in Fas expression (C) according to the body weight in liver tissues of obesity model mice (HFD), and the proportions (D) of Fas-expressing cells: NCD = normal control diet group; HFD = high fat diet group; WAT = white adipose tissue; and Liver = liver tissue.
FIG. 9 illustrates the results of confirming whether FBP-8 binds to Fas in the white adipose tissue and liver tissue sections of obesity model mice (HFD).
FIG. 10 illustrates the results of injecting fluorescently labeled FBP-8 into obesity model mice (HFD) by subcutaneous injection (A) or intravenous injection (B) and confirming the fluorescence distribution thereof in each organ after 24 hours and 48 hours: Mock = non-administration group (no peptide administration); CtrFBP = negative control (control peptide administration); and FBP-8 = FBP-8 administration group.
FIG. 11 illustrates the results of confirming the fluorescence signals of FBP-8 in the liver tissue sections of obesity model mice into which FBP-8 is injected by intravenous injection.
FIG. 12 illustrates the results of injecting fluorescently labeled FBP-8 into normal body weight mice by intravenous injection (IV) or subcutaneous injection (SC), and confirming the fluorescence distribution thereof in each organ after 12 hours.
FIG. 13 schematically illustrates the process of an animal experiment according to an exemplary embodiment of the present invention.
FIG. 14 illustrates the results of confirming the proportion of dead cells by TUNEL staining in liver tissue sections of obesity model mice into which FBP-8 is injected by intravenous injection.
FIG. 15 illustrates the results of confirming the proportion of dead cells by TUNEL staining in white adipose tissue sections of obesity model mice into which FBP-8 is injected by intravenous injection.
FIG. 16 illustrates the results of confirming the expression levels of F4/80 and CD11c which are pro-inflammatory macrophage-labeled genes in white adipose tissues of obesity model mice into which FBP-8 is injected by intravenous injection.
FIG. 17 illustrates the results of confirming the crown-like structure (CLS) area in white adipose tissues of obesity model mice into which FBP-8 is injected by intravenous injection.
FIG. 18 illustrates the results of confirming the expression levels of inflammatory response-related genes in white adipose tissues of obesity model mice into which FBP-8 is injected by intravenous injection.
FIG. 19 illustrates the results of measuring the levels of pro-inflammatory cytokines in the blood of obesity model mice into which FBP-8 is injected by intravenous injection.
FIG. 20 illustrates the results of confirming the expression levels of F4/80, CD11c, and CD206 in liver tissues of obesity model mice into which FBP-8 is injected by intravenous injection.
FIG. 21 illustrates the results of confirming the expression levels of inflammatory response-related genes in liver tissues of obesity model mice into which FBP-8 is injected by intravenous injection.
FIG. 22 illustrates the results of evaluating glucose resistance in obesity model mice into which FBP-8 is injected by intravenous injection.
FIG. 23 illustrates the results of evaluating insulin sensitivity in obesity model mice into which FBP-8 is injected by intravenous injection.
FIG. 24 illustrates the results of confirming the daily food intake (A) and body weight change (B) in the process of administering FBP-8 to obesity model mice by intravenous injection.
FIG. 25 illustrates the results of confirming the expression levels of stearoyl-CoA desaturase (SCD-1) which is an adipose-producing gene in liver tissues of obesity model mice into which FBP-8 is injected by intravenous injection.
FIG. 26 illustrates the results of measuring fatty acid and insulin concentrations in the blood of obesity model mice into which FBP-8 is injected by intravenous injection.
FIG. 27 illustrates the results of measuring the concentrations of triglyceride (TG) in liver tissues of obesity model mice into which FBP-8 is injected by intravenous injection.
FIG. 28 illustrates the results of confirming the liver tissue morphology and weight of obesity model mice into which FBP-8 is injected by intravenous injection.
FIG. 29 illustrates the results of confirming the degree of liver damage in liver tissue sections of obesity model mice into which FBP-8 is injected by intravenous injection.
FIG. 30 illustrates the results of measuring the alanine aminotransferase (ALT) level, which is a liver function index, in the blood of obesity model mouse into which FBP-8 is injected by intravenous injection.
FIG. 31 illustrates the results of confirming the proportion of dead cells by TUNEL staining in liver tissue sections of obesity model mice into which FBP-7 is injected by subcutaneous injection: NCD = normal group (normal control diet group); Mock = non-administration group (no peptide administration); CtrFBP = negative control (control peptide administration); Saxenda = positive control (liraglutide administration); and FBP-7 = FBP-7 administration group.
FIG. 32 illustrates the results of confirming the expression levels of inflammatory response-related macrophage-labeled genes in liver tissues of obesity model mice into which FBP-7 is injected by subcutaneous injection.
FIG. 33 illustrates the results of confirming the expression levels of inflammatory response-related genes in liver tissues of obesity model mice into which FBP-7 is injected by subcutaneous injection.
FIG. 34 illustrates the results of confirming the expression levels of Fas genes in liver tissues of obesity model mice into which FBP-7 is injected by subcutaneous injection.
FIG. 35 illustrates the results of measuring the levels of pro-inflammatory cytokines in the blood of obesity model mice into which FBP-7 is injected by subcutaneous injection.
FIG. 36 illustrates the results of evaluating glucose resistance in obesity model mice into which FBP-7 is injected by subcutaneous injection.
FIG. 37 illustrates the results of measuring the body weight of obesity model mice into which FBP-7 is injected by subcutaneous injection over time.
FIG. 38 illustrates the results of confirming the food intake of obesity model mice into which FBP-7 is injected by subcutaneous injection over time.
FIG. 39 illustrates the results of confirming the liver tissue morphology and weight of obesity model mice into which FBP-7 is injected by subcutaneous injection.
FIG. 40 illustrates the results of confirming the degree of liver damage in liver tissue sections of obesity model mice into which FBP-7 is injected by subcutaneous injection.
FIG. 41 illustrates the results of confirming the expression levels of PPAR-γ which is a gene involved in fat accumulation, in liver tissues of obesity model mice into which FBP-7 is injected by subcutaneous injection.
FIG. 42 illustrates the results of confirming the level of triglyceride in liver tissues of obesity model mice into which FBP-7 is injected by subcutaneous injection.
FIG. 43 illustrates the results of measuring fatty acid and insulin concentrations in the blood of obesity model mice into which FBP-7 is injected by subcutaneous injection.
FIG. 44 illustrates the results of measuring the ALT level, which is a liver function index, in the blood of obesity model mouse into which FBP-7 is injected by subcutaneous injection.

### [Modes of the Invention]

Hereinafter, the present invention will be described in more detail through Examples. These Examples are provided only for more specifically describing the present invention, and it will be obvious to a person with ordinary skill in the art to which the present invention pertains that the scope of the present invention is not limited by these Examples according to the gist of the present invention.

### Experimental methods

### 1. Peptide

Peptides were used by synthesizing the following sequences at PEPTRON (Daejeon, Republic of Korea) and then dissolving the peptides in PBS with a pH of 7.4 in a freeze-dried state. The first peptide is disclosed in a related art document (Proc Natl Acad Sci U S A. 2004 Apr 27;101(17):6599-604).
Fas Blocking Peptide (FBP or FBP-8): YCDEHFCY (SEQ ID NO: 11)
Fas Blocking Peptide 7 (FBP7): YCDEHF-Y (SEQ ID NO: 10)
Fas Blocking Peptide A (FBPA): YCDEHFAY (SEQ ID NO: 12)
Control Peptide (CTRP): YCNSTVCY (SEQ ID NO: 13)

### 2. Cell culture

A Jurkat cell line which is a human blood cancer cell line and a 3T3L1 cell line which is a murine adipocyte were purchased from ATCC (USA), and the Jurkat cell line was cultured in an RPMI culture medium containing 10% fetal bovine serum (FBS), 1% penicillin, 1% streptomycin, and 25 mM glucose.

The 3T3L1 cell line was cultured in a DMEM culture medium containing 10% FBS, 1% penicillin, 1% streptomycin, and 25 mM glucose. The 3T3L1 cell line was aliquoted into the above DMEM culture solution at a concentration of 2 x 10⁵ cells/ml to induce adipocyte maturation for 3 days, and then replaced with a culture solution containing insulin to induce adipocyte maturation again for 3 days. Thereafter, the 3T3L1 cell line was further cultured in the above DMEM culture solution for 2 days to achieve complete maturation of adipocytes.

### 3. Cytotoxicity and apoptosis experiments

The cytotoxicity experiments of the peptides described in 1. above were performed by a CCK-8 analysis kit (Dojindo Laboratories, Japan). The 3T3L1 cell line was treated with peptides at various concentrations (uM) and cytotoxicity was analyzed after 24 hours.

The 3T3L1 cell line was treated with a Fas ligand (FasL) at a concentration of 500 ng/ml to induce apoptosis, and FBP signal blocking peptides (FBP-8, FBP-A and FBP-7) and CtrFBP bound to Alexa647 were treated, respectively. After 4 hours of peptide treatment, cells were washed with DPBS and treated with FITC-bound annexin V (BD Pharmingen) to analyze the apoptosis inhibitory effect by a flow cytometer.

### 4. Animal experiments

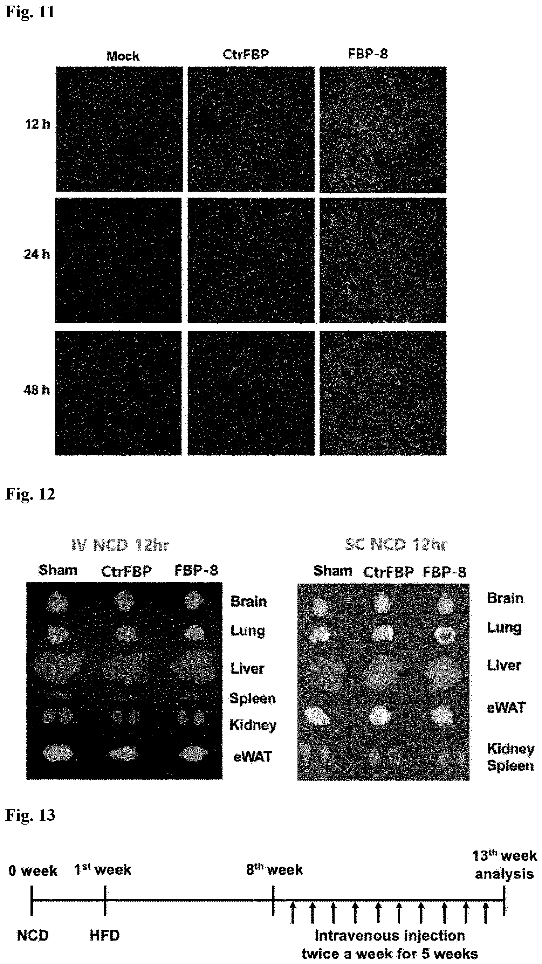

All animal experiments performed for the present invention were approved by the Institutional Animal Care and Use Committee of Hanyang University. Six-week-old C57BL/6 mice were fed a high fat diet for eight weeks to produce obesity model mice, and when the mice had a weight in a range of 42 to 44 g, the mice were randomly classified into experimental groups. Thereafter, depending on the experimental group, 60 µg/one time of FBP8 were administered by intravenous injection twice a week for a total of 5 weeks (FIG. 13), or FBP7 was administered by subcutaneous injection twice a day.

### 5. Tissue immunological analysis

After the animal experiment was completed, mice were sacrificed to isolate liver tissues and white adipose tissues and prepare tissue sections. The prepared liver tissue and white adipose tissue sections were treated with an antigen retrieval buffer at 95°C for 25 minutes and then cooled at room temperature. Thereafter, the tissue sections were treated with Tris Based Saline + Tween20 (TBST) containing 1% bovine serum albumin (BSA), 10% goat serum and 0.05% Tween 20 to undergo a blocking process, and were reacted with a Fas receptor-specific primary antibody at 4°C for 18 hours. Thereafter, tissue sections were washed with TBST and reacted with a FITC-bound secondary antibody at room temperature for 2 hours.

In addition, in order to examine the tissue-binding ability of the peptide, the peptide to which Alexa 488 was bound was reacted with the primary antibody at 4°C for 18 hours. The nuclei were stained with Hoechst 33342 (GE Healthcare), washed with TBST, and then confirmed.

The fluorescence signal of each tissue section was analyzed under a TCS-SP5 confocal microscope (Leica, Germany).

### 6. Analysis of delivery of peptide to tissues

100 µg of an Alexa 647-bound peptide was administered by intravenous injection into obesity model mice, and after 12, 24, and 48 hours, the tissues were isolated, and the fluorescence signals in each tissue were analyzed with an image station fluorescence analyzer (Carestream). After the fluorescence signal analysis, the relative fluorescence intensity of each tissue was compared using an ImageJ program provided by NIH.

In addition, 100 µg of Alexa 488-bound FBP was administered by intravenous injection into obesity model mice to confirm whether cell units of peptides were delivered to liver tissues, and frozen tissue sections were produced by isolating the liver tissues after 12, 24, and 48 hours. Thereafter, the nuclei were stained with Hoechst 33342, and the fluorescence signals were analyzed under a TCS-SP5 confocal microscope.

### 7. Histological analysis and TUNEL analysis

Liver tissue and white adipose tissue sections were stained with hematoxylin & eosin and then histologically analyzed under an optical microscope.

Furthermore, a terminal deoxynucleotidyl transferase dUTP nick end labeling (TUNEL) analysis for each tissue was performed with an in situ cell death detection kit (Millipore, USA). Thereafter, the fluorescence signals were analyzed under a TCS-SP5 confocal microscope by staining the nuclei with Hoechst 33342, and the fluorescence signals of each cell were analyzed with the Image J program.

### 8. Gene expression level analysis

mRNA was extracted from liver tissues and white adipose tissues using RNAiso (Takara, Japan), and cDNAwas synthesized with an iscript cDNA synthesis kit (Bio-Rad Laboratories, USA). Thereafter, a gene expression level was analyzed by the 7500 Fast Real-time PCR system (Applied Biosystems, USA). The expression level of each gene was normalized with the expression level of a GAPDH gene, and then calculated as a relative gene expression level with respect to the control.

### 9. Analysis of glucose intolerance and insulin resistance

For a glucose intolerance analysis, a dose of 2 g/kg of glucose was injected by intraperitoneal injection into each experimental group maintained in a fasting state for 12 hours, and blood glucose levels were measured after 30, 60 and 120 minutes by Accu-check (Roche, USA).

Insulin resistance was analyzed by measuring blood glucose levels 30 minutes, 60 minutes and 120 minutes after a dose of 0.75 U/kg of insulin was injected by intraperitoneal injection into each experimental group maintained in a fasting state for 4 hours.

### 10. Other analyses

The cytokine levels of serum and tissues were analyzed with an ELISA kit (eBioscience, USA), and the triglyceride levels of liver tissues were confirmed with a triglyceride assay kit (Cayman, USA).

### 11. Statistical analysis

For the experimental results, a non-parametric Mann-Whitney U analysis was used when there were two different experimental groups, and statistical significance was confirmed by a one-way ANOVA analysis when there were three or more experimental groups. A GraphPad Prism 5 program was used for statistical analysis. A P value of 0.05 or less was determined to have statistical significance.

### Experimental Results

### 1. Confirmation of the binding force of Fas blocking peptides to Fas-expressing cell lines and the effects of controlling of Fas signals

In addition to the previously revealed Fas signaling inhibitory peptide (YCDEHFCY, FBP-8), two types of peptides FBP-A (YCDEHFAY) and FBP-7 (YCDEHFY) with modified sequences were prepared, and their specific binding force to Fas-expressing cell lines and effects of controlling of signals were confirmed.

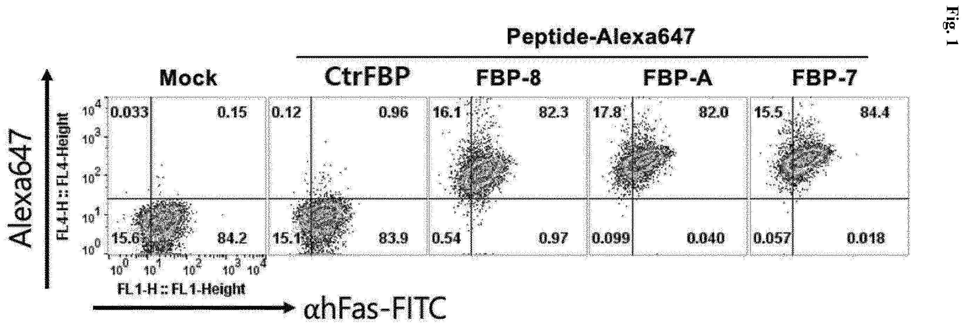

A Jurkat cell line, in which Fas is always expressed, was treated with an FITC-labeled Fas antibody and treated with Alexa647-labeled FBP-8, FBP-A, FBP-7 or control peptide (YCNSTVCY; CtrFBP). Thereafter, as a result of analyzing fluorescence signals, it could be seen that the control peptide hardly bound to Jurkat cells since an Alexa647 signal was hardly confirmed in a control (CtrFBP; a control peptide-treated group), and it could be confirmed that FBP-8-, FBP-A-, and FBP-7-treated groups bound to Jurkat cells at similar levels (FIG. 1).

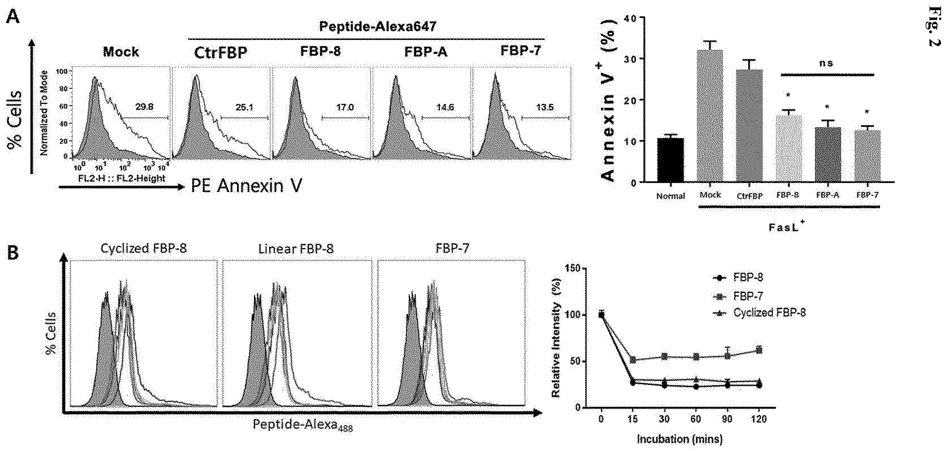

In addition, as a result of confirming the apoptosis levels after treating Jurkat cell line with a Fas ligand (FasL) and an inhibitory peptide against Fas signaling, all of FBP-8, FBP-A and FBP-7 exhibited an effect of inhibiting Fas signaling at similar levels. Specifically, it could be confirmed that the negative control (CtrFBP) had an apoptosis level similar to that of an untreated group (Mock; experimental group not treated with any peptide) (31±2.32%and 29±3.24%, respectively), but FBP-8, FBP-A and FBP-7-treated groups exhibited an apoptosis level of 17±4.11%, 15±5.26%, and 14±3.22%, respectively, and thus apoptosis was effectively inhibited compared to the untreated group (Mock) (FIG. 2A). Furthermore, as a result of confirming the serum stability of cyclic FBP-8, linear FBP-8 and FBP-7, it could be seen that FBP-7 was the best (FIG. 2B).

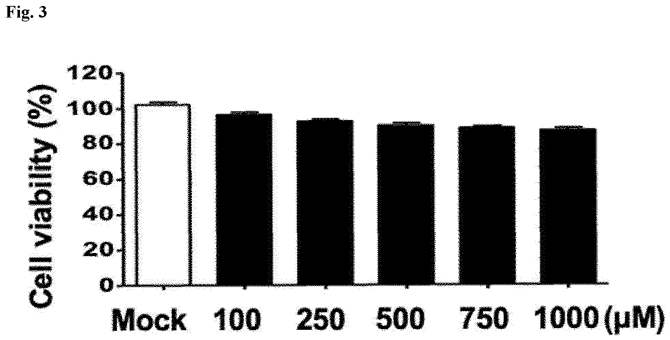

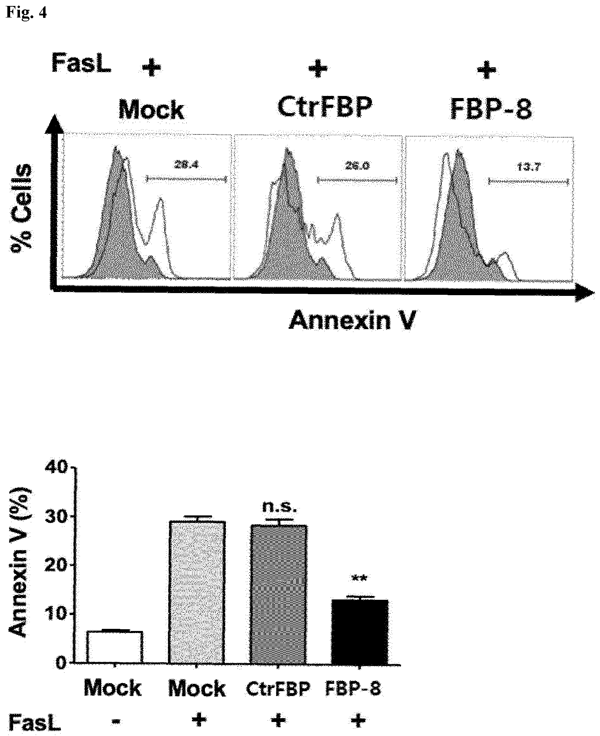

Further, the same experiment was performed on a 3T3L1 cell line having a Fas signaling pathway different from that of the Jurkat cell line. It could be confirmed that when differentiated 3T3L1 cell lines were treated with FBP-8 at various concentrations from 100 µM to 1000 µM, little cytotoxicity was observed (FIG. 3), and a combined treatment with the Fas ligand and FBP-8 remarkably inhibited apoptosis compared to the untreated group (Mock+) and the negative control (CtrFBP). The apoptosis levels in each experimental group were 25±3.31%, 23±5.12% and 14±2.98%, respectively (FIG. 4).

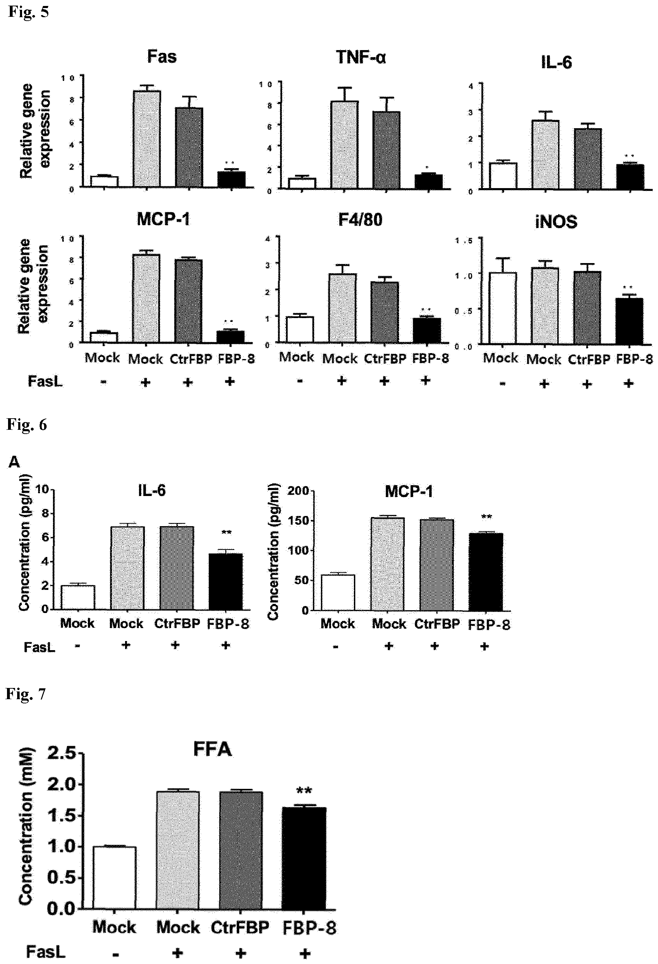

The excellent Fas signaling inhibitory effect of FBP-8 could also be confirmed by the expression levels of inflammation-related genes. It could be observed that when differentiated 3T3L1 cell lines were treated with the Fas ligand and FBP-8 in combination, the expressions of the inflammation-related genes Fas, TNF-α, monocyte chemoattractant protein-1 (MCP-1), F4/80, IL-6 and an inducible nitric oxide synthase (iNOS) were remarkably reduced (FIG. 5).

It could be seen that inhibition of the expression of these inflammation-related genes led to a decrease in production of inflammation-related cytokines and the FBP-8 treatment reduces the concentrations of IL-6 and MCP-1 in the cell culture solution (FIG. 6). These results mean that the FBP-8 treatment can effectively inhibit a Fas-derived inflammation-related signaling pathway.

In addition, the inflammation of adipocytes led to apoptosis, so ultimately, fatty acids (FFA) intracellularly stored were released extracellularly, but the FBP-8 treatment also significantly inhibited the extracellular release of fatty acids (FIG. 7).

### 2. Evaluation of specific binding ability of Fas-binding peptide to liver tissues and white adipose tissues in non-alcoholic steatohepatitis animal model

When a normal-weight mouse is fed a high fat diet, the mouse becomes an obesity model mouse, and in this case, the expression level of Fas increases in liver tissues or white adipose tissues. Specifically, as a result of confirming the expression level of Fas in the white adipose tissue (WAT) and liver tissue of the high fat diet (HFD) group, it could be seen that the level was increased by 4-fold and 3-fold, respectively compared to that of the normal control diet (NCD)(FIGS. 8A and 8B). It could be seen that in particular, in the case of the high fat diet (HFD) group, the expression level of Fas also increased in proportion to the increase in body weight. (FIG. 8C). This increase in expression could be further confirmed by an increase in proportion of Fas-expressing cells in the actual tissue, and the proportion of Fas-expressing cells in the white adipose tissue and liver tissue of the high fat diet (HFD) group increased to 70% and 75%, respectively, compared to the normal diet group (NCD)(FIG 8D).

In addition, when liver tissue sections and white adipose tissue sections isolated from the high fat diet (HFD) group were treated with an Fas-specific antibody and fluorescently labeled FBP-8, the stained sites coincided (FIG. 9), meaning that FBP-8 effectively binds to Fas overexpressed by obesity.

Based on the Fas-specific binding ability of FBP-8 confirmed by the experiments, it was confirmed whether FBP-8 could be specifically delivered to liver tissues or adipose tissues when systemically delivered by intravenous injection or subcutaneous injection. Fluorescently labeled FBP-8 was injected by intravenous injection or subcutaneous injection into obesity model mice in the high fat diet group, and after 24 and 48 hours, the fluorescence distribution was investigated by isolating each organ.

As a result, it could be seen that FBP-8 injected by subcutaneous injection was specifically delivered to the liver tissue compared to the negative control (CtrFBP) and remained in the liver tissue until 48 hours or later (FIG. 10A). FBP-8 injected by intravenous injection was specifically delivered to the liver tissue and white adipose tissue (FIG. 10B). As a result of observing the liver tissue sections, it was found that FBP-8 injected by intravenous injection was effectively delivered to the liver tissue from 12 hours after the injection, and remained in the liver tissue until 48 hours after the injection (FIG. 11).

It could be confirmed that when FBP-8 was injected into normal mice by intravenous injection (IV) or subcutaneous injection (SC), a significant fluorescence signal could not be observed in the liver tissue, indicating that FBP-8 was not delivered to the liver (FIG. 12).

Through the results in FIGS. 10 to 12, it can be seen that the expression of Fas receptors in the liver tissue and white adipose tissue is increased by obesity, and FBP-8 specifically binds to Fas-expressing liver tissues and white adipose tissues.

### 3. Evaluation of therapeutic efficacy for non-alcoholic steatohepatitis, insulin resistance and metabolic diseases by systemically delivered FBP

Since it was confirmed in FIGS. 10 and 11 that FBP-8 injected by intravenous injection was delivered to the liver tissues and white adipose tissues of obesity model mice, it was confirmed whether, by the control of Fas signaling caused by systemic delivery of FBP, it was possible to obtain the effects of alleviating inflammation and inhibiting apoptosis in liver tissues and white adipose tissues.

Obesity model mice were produced by feeding normal mice a high fat diet for 8 weeks, and FBP-8 was administered by intravenous injection twice a week for a total of 5 weeks (FIG. 13). After five weeks, the obesity model mice were sacrificed to isolate white adipose tissues and liver tissues and analyze the proportion of dead cells by TUNEL staining.

As a result of the analysis, it was confirmed that in the case of the liver tissue, the proportion of dead cells in the non-administration group (Mock) and the negative control (CtrFBP) was 19±5.28% and 17±7.98%, respectively, whereas the proportion of dead cells in the FBP-8-treated group was shown to be 9±2.37%, and thus was significantly reduced (FIG. 14).

It could be seen that in the case of the white adipose tissue, the proportion of dead cells in the non-administration group (Mock) and the negative control (CtrFBP) was 55±3.42% and 51±2.98%, respectively, whereas the proportion of dead cells in the FBP-8-treated group was shown to be 31±2.37%, and thus was remarkably reduced (FIG. 15).

The effect of controlling Fas signaling by FBP-8 inhibits not only apoptosis but also an inflammatory response, and as a result of confirming the expression levels of F480 and CD11c, which are pro-inflammatory macrophage markers in white adipose tissue, it could be seen that the expression level was significantly reduced in the FBP-8-treated group compared to the negative control (CtrFBP), and thus, the inflammatory response was inhibited in white adipose tissues (FIG. 16).

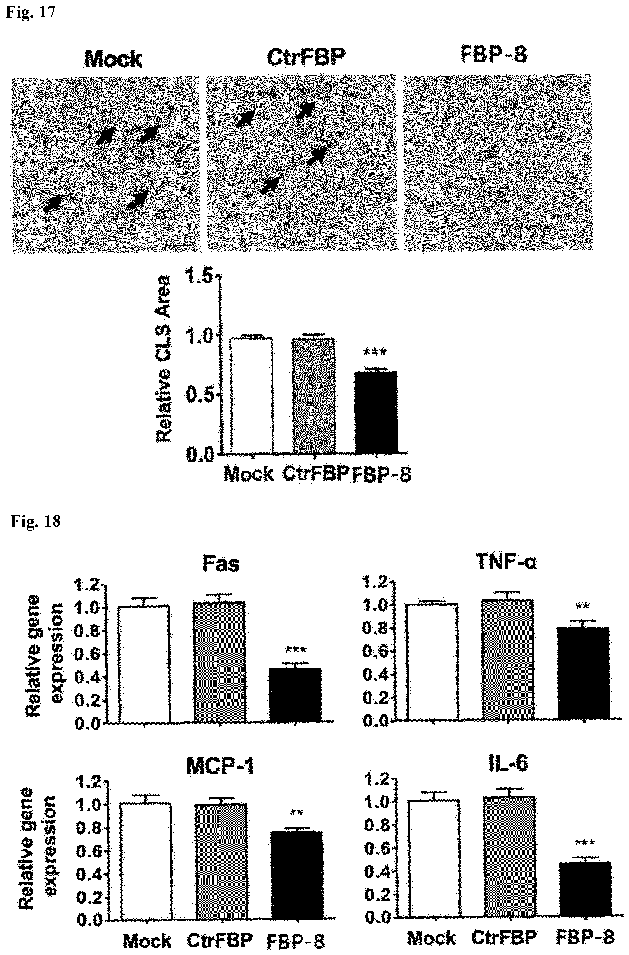

Further, a crown-like structure (CLS) formed by gathering macrophages around adipocytes in white adipose tissues, was also remarkably reduced in the FBP-8-treated group compared to the negative control (CtrFBP)(FIG 17), and in white adipose tissues, the expression of inflammation-related genes such as Fas, TNF-α, MCP-1, and IL-6 was also reduced in the FBP-8-treated group compared to the negative control (CtrFBP)(FIG 18).

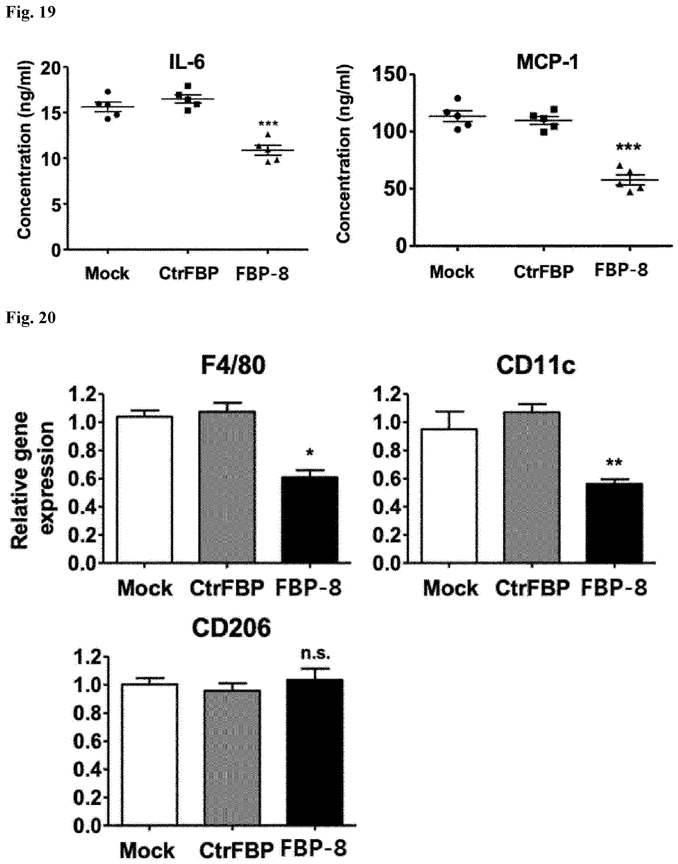

Although in an obese state, pro-inflammatory cytokines produced in white adipose tissues are delivered to liver tissues through blood to induce inflammation, it could be confirmed that the concentrations of IL-6 and MCP-1 in blood decrease in the FBP-8-treated group (FIG. 19).

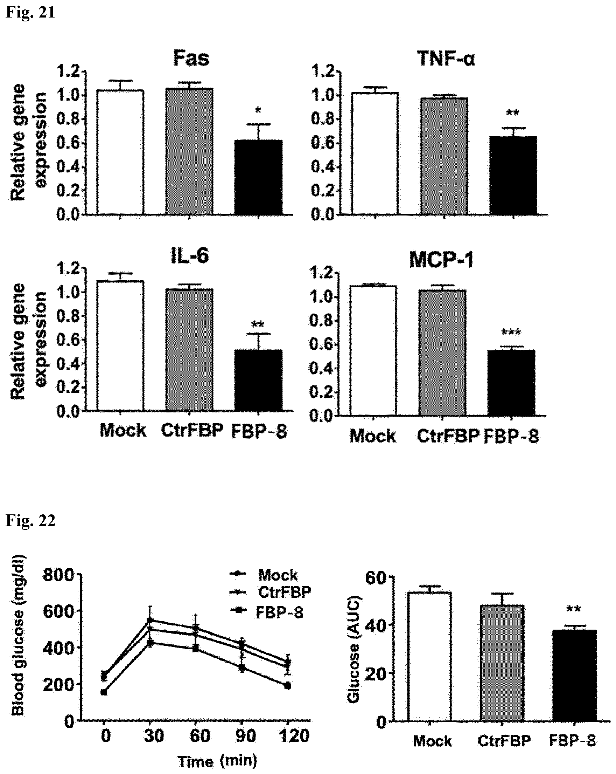

An anti-inflammatory effect similar to that of white adipose tissues could be confirmed even in liver tissues, and the expression levels of F480 and CD11c, which are pro-inflammatory macrophage markers, decreased in the FBP-8-treated group compared to the negative control (CtrFBP), and there was no significant change in the expression level of CD206 which is an anti-inflammatory macrophage marker (FIG. 20). The expression levels of inflammation-related genes such as Fas, TNF-α, MCP-1, and IL-6 were also significantly reduced in the FBP-8-treated group compared to the negative control (CtrFBP)(FIG 21).

The systemic delivery of FBP-8 also improved glucose metabolism disorders according to an increase in insulin resistance in obesity model mice. Specifically, it could be observed that in a glucose tolerance test (GTT) and an insulin tolerance test (ITT), the negative control (CtrFBP) showed changes in blood glucose levels similar to those in the non-administration group (Mock), but the FBP-8-treated group improved glucose metabolism compared to the negative control (CtrFBP)(FIGS. 22 and 23).

There was no significant change in daily food intake in each experimental group (FIG. 24A), but the FBP-8-treated group tended to have slower body weight gain compared to the negative control (CtrFBP)(FIG 24B).

The systemic delivery of FBP-8 alleviated inflammation, inhibited apoptosis, and improved glucose metabolism disorders in liver tissues and white adipose tissues, and as a result, non-alcoholic steatohepatitis caused by obesity was also improved.

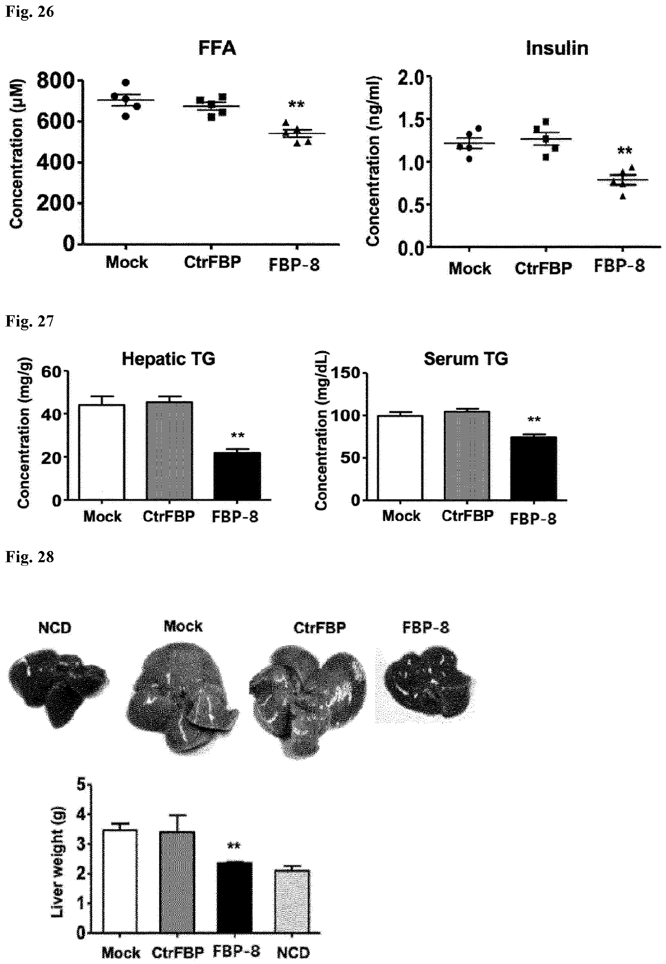

Specifically, as a result of confirmation in the liver tissues of obesity model mice, the expression level of a stearoyl-CoA desaturase (SCD-1), which is an adipose-producing gene, in the FBP-8-treated group was reduced by about 30±5.32% compared to the negative control (CtrFBP)(FIG 25), and the concentrations of fatty acids and insulin in blood was also decreased by about 20% compared to the negative control (CtrFBP)(FIG 26). In the case of the FBP-8-treated group, the concentration of triglyceride (TG) was reduced by about 50% and about 25% in the liver tissue and blood, respectively (FIG. 27).

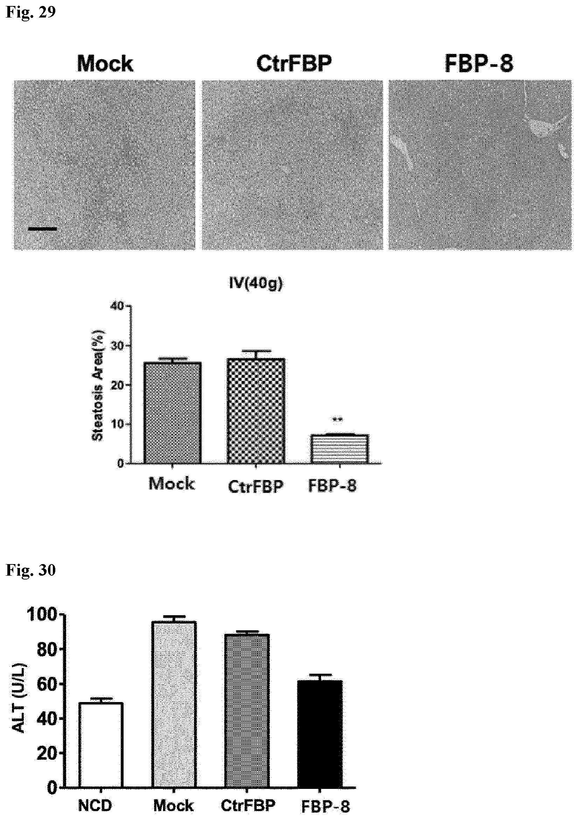

In addition, in the FBP-8-treated group, the size of the liver tissue was smaller and the weight was lighter than the negative control (CtrFBP)(FIG 28), and through a histological analysis of liver tissue sections, it could be observed that damage to the liver tissue in the FBP-8-treated group was inhibited (FIG. 29). Even from the results of measuring a blood alanine aminotransferase (ALT) level, which is a major index for evaluating the function of liver tissue, it could be confirmed that the ALT level in the FBP-8-treated group was reduced by 30% or more compared to the negative control (CtrFBP)(FIG 30).

### 4. Evaluation of therapeutic efficacy for non-alcoholic steatohepatitis, insulin resistance and metabolic diseases by FBP-7 systemically delivered by subcutaneous injection

Obesity model mice were produced by feeding normal mice a high fat diet for 8 weeks, and FBP-7 was administered by subcutaneous injection twice a day for a total of 3 weeks. A control peptide was administered as the negative control (CtrFBP), and liraglutide (trade name: Saxenda) commercially available as an obesity therapeutic agent was administered as the positive control.

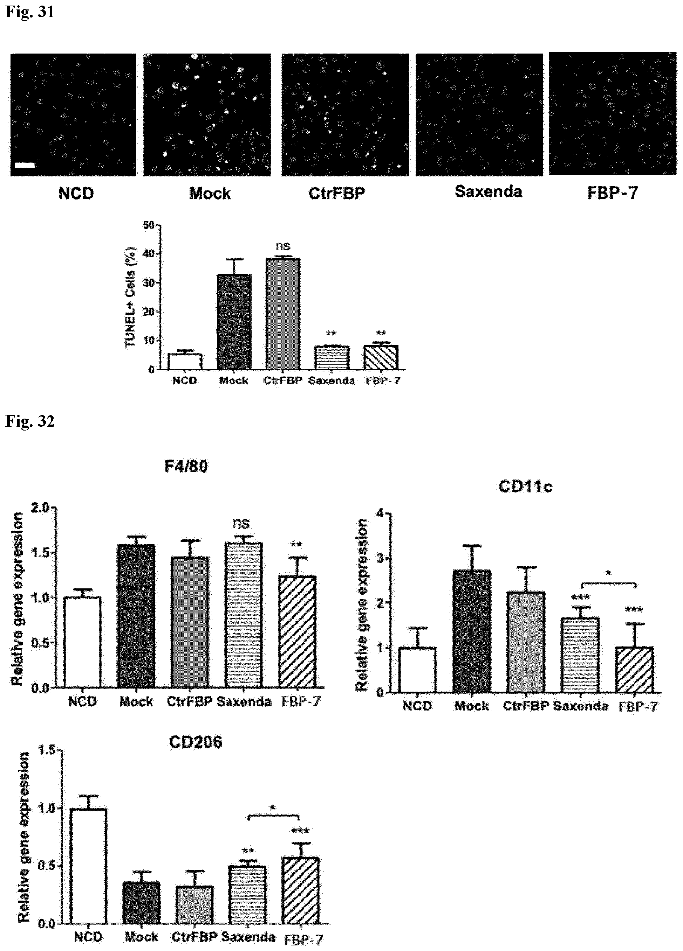

After three weeks, the obesity model mice were sacrificed to isolate liver tissues and analyze the proportion of dead cells by TUNEL staining. As a result, the proportion of dead cells in the FBP-7-administered group was shown to be 10±2.45%, which was remarkably lower than that in the non-administered group (Mock), and the negative control (CtrFBP), and the FBP-7-administered group exhibited a level similar to that (11±4.89%) of a liraglutide-administered group which is a positive control (FIG. 31).

The inflammatory response in the liver tissue was also inhibited by the administration of FBP-7. Compared to the negative control (CtrFBP), the expression levels of F480 and CD11c, which are pro-inflammatory macrophage marker genes, were decreased and the expression level of CD206, which is an anti-inflammatory macrophage marker, was increased remarkably in the FBP-7-administered group (FIG. 32). Specifically, the expression level of CD206 was increased more in the FBP-7-administered group than in the liraglutide-administered group, and the expression level of F4/80 was significantly decreased.

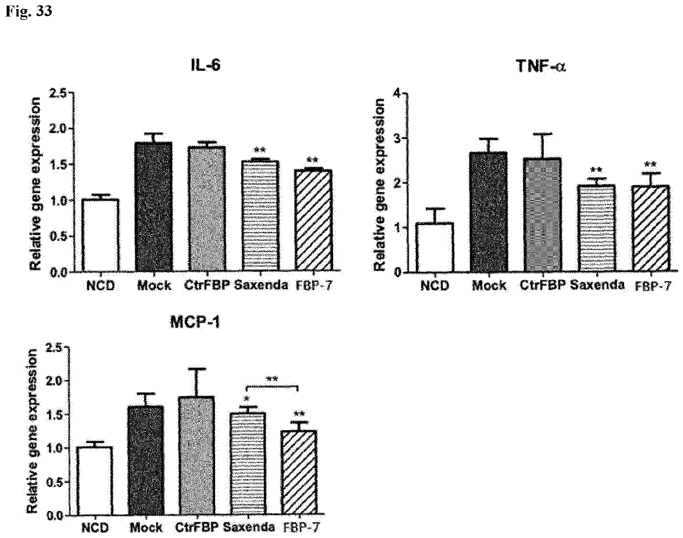

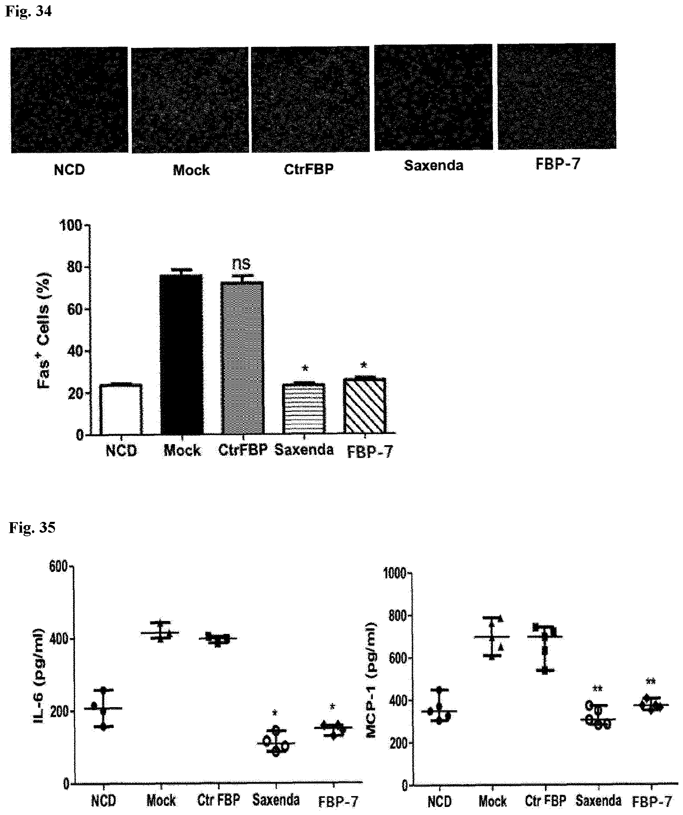

The expression levels of the inflammation-related genes TNF-α, MCP-1 and IL-6 were also decreased in the FBP-7-administered group compared to the negative control group (CtrFBP)(FIG 33), and the expression of Fas, which is a major marker of inflammation, was also remarkably reduced in the liver tissue in the FBP-7-treated group (FIG. 34). By inhibiting the inflammatory response in such liver tissue, the blood levels of the pro-inflammatory cytokines IL-6 and MCP-1 were also reduced (FIG. 35).

The effect of inhibiting the inflammatory response by systemic delivery of FBP-7 also led to an effect of improving glucose metabolism disorders. In a glucose resistance evaluation experiment, the FBP-7-administered group showed an excellent blood glucose increase-slowing effect and normal blood glucose recovery compared to the negative control (CtrFBP) to alleviate glucose metabolism disorders, and showed an improvement effect which is similar to that of liraglutide (FIG. 36).

Due to such an improvement in glucose metabolism disorders, the body weight gain in the FBP-7-administered group was remarkably suppressed compared to the negative control (FIG. 37), and the FBP-7-administered group also showed a pattern of a slight decrease in food intake (FIG. 38).

The effects of FBP-7 administration on alleviation of inflammation and inhibition of apoptosis in the liver tissues ultimately improved non-alcoholic steatohepatitis caused by obesity.

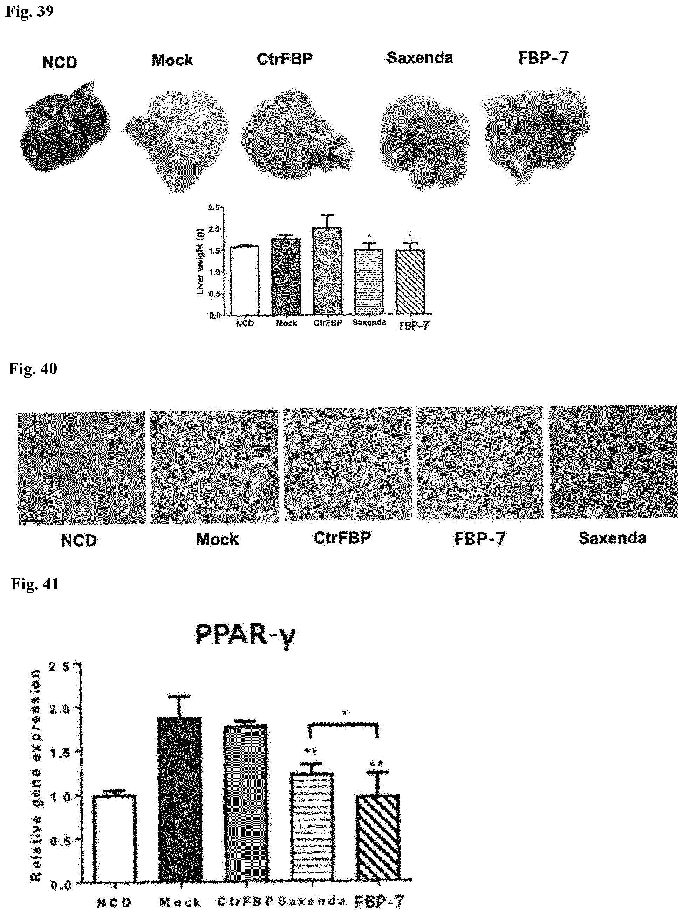

Livers in the FBP-7-administered group had reduced tissue weight compared to the negative control (CtrFBP)(FIG 39), and a histological analysis of liver tissue sections also showed an effect of inhibiting tissue damage at a level similar to that of the liraglutide-administered group (FIG. 40).

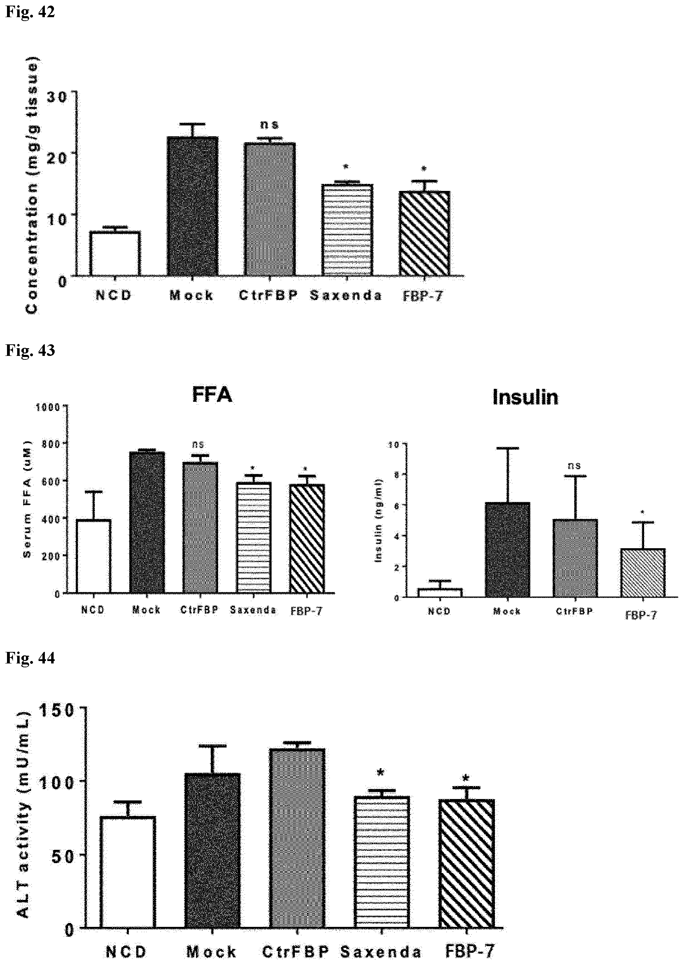

Furthermore, compared to the negative control (CtrFBP), the expression level of PPAR-γ, which is a gene involved in fat accumulation in the liver, was reduced in the FBP-7-administered group, and this expression-reducing effect was even remarkably better than that of the liraglutide-administered group which is a positive control (FIG. 41). In addition, compared to the negative control (CtrFBP), it can be confirmed that the triglyceride level in the liver tissue and blood fatty acid concentration were also significantly reduced in the FBP-7-administered group (FIGS. 42 and 43), and a blood alanine aminotransferase (ALT) level, which is a major index for evaluating the function of liver tissue, was significantly reduced in a FBP-7-administered group (FIG. 44).

The inhibitory peptide against Fas signaling of the present invention is specifically delivered to liver tissues and white adipose tissues when injected by intravenous injection into obesity model mice and specifically delivered to liver tissues when injected by subcutaneous injection into the obesity model mice. The inhibitory peptide binds to Fas, which is abundantly expressed in the liver and adipose tissues during obesity after injection, and then inhibits an inflammatory response and apoptosis by blocking downstream signaling, and as a result, body weight gain, fat accumulation, and the like can be inhibited.

## Claims

1. A pharmaceutical composition comprising an inhibitory peptide against Fas signaling, comprising an amino acid sequence represented by the following General Formula 1 as an active ingredient for prevention or treatment of obesity, fatty liver, or steatohepatitis,
[General Formula I] Xaa₁-Cys-Asp-Glu-His-Phe-Xaa₂-Xaa₃,
in the general formula, Xaa₁ and Xaa₃ are each independently absent or any amino acid, and Xaa₂ is absent or selected from the group consisting of Ala, Gly, Val, Leu, Ile, Met, Pro, Ser, Cys, Thr, Asn, and Gln.

2. The pharmaceutical composition of claim 1, wherein the inhibitory peptide against Fas signaling comprises an amino acid sequence represented by the following General Formula I,
[General Formula I] Xaa₁-Cys-Asp-Glu-His-Phe-Xaa₂-Xaa₃,
in the general formula, Xaa₁ and Xaa₃ are each independently absent or selected from the group consisting of Tyr, Phe, and Trp, and Xaa₂ is absent or selected from the group consisting of Gly, Ala, Ser, Thr, and Cys.

3. The pharmaceutical composition of claim 1, wherein the inhibitory peptide against Fas signaling comprises an amino acid sequence represented by the following General Formula I,
[General Formula I] Xaa₁-Cys-Asp-Glu-His-Phe-Xaa₂-Xaa₃,
in the general formula, Xaa₁ and Xaa₃ are each independently selected from the group consisting of Tyr, Phe, and Trp, and Xaa₂ is selected from the group consisting of Gly, Ala, Ser, Thr, and Cys.

4. The pharmaceutical composition of claim 1, wherein the fatty liver is non-alcoholic fatty liver.

5. The pharmaceutical composition of claim 1, wherein the pharmaceutical composition is administered via a route selected from the group consisting of intravenous, subcutaneous, and oral routes.

6. The pharmaceutical composition of claim 1, wherein the pharmaceutical composition is administered in the form of an injection.

7. The pharmaceutical composition of claim 1, wherein the amino acid sequence of General Formula I comprises a sequence selected from the group consisting of SEQ ID NOS: 1 to 12.

8. The pharmaceutical composition of claim 6, wherein the amino acid sequence of General Formula I comprises a sequence selected from the group consisting of SEQ ID NOS: 7 to 12.

9. The pharmaceutical composition of claim 7, wherein the amino acid sequence of General Formula I comprises a sequence selected from the group consisting of SEQ ID NOS: 10 to 12.

10. A pharmaceutical composition comprising an inhibitory peptide against Fas signaling, comprising an amino acid sequence represented by the following General Formula 1 as an active ingredient for prevention or treatment of obesity, fatty liver, or steatohepatitis,
[General Formula I] Xaa₁-Cys-Asp-Glu-His-Phe-Xaa₂-Xaa₃,
in the general formula, Xaa₁ and Xaa₃ are each independently absent or any amino acid, and Xaa₂ is absent or selected from the group consisting of Ala, Gly, Val, Leu, Ile, Met, Pro, Ser, Cys, Thr, Asn, and Gln.
